(19) Europäisches Patentamt
European Patent Office
Office européen des brevets

(11) Publication number: **0 208 617 B1**

(12) **EUROPEAN PATENT SPECIFICATION**

(45) Date of publication of the patent specification: **26.09.90**

(51) Int. Cl.[5]: **A61K 9/22**, A61K 47/00

(21) Application number: **86401501.1**

(22) Date of filing: **04.07.86**

(54) **Controlled drug device and process for catalysing the erosion of an acid labile polymer.**

(30) Priority: **08.07.85 US 752436**

(43) Date of publication of application:
**14.01.87 Bulletin 87/3**

(45) Publication of the grant of the patent:
**26.09.90 Bulletin 90/39**

(84) Designated Contracting States:
**CH DE FR GB IT LI NL**

(56) References cited:
**EP-A- 0 052 916**
**EP-A- 0 100 424**

(73) Proprietor: **MERCK & CO. INC., 126, East Lincoln Avenue P.O. Box 2000, Rahway New Jersey 07065-0900(US)**

(72) Inventor: **Himmelstein, Kenneth J., 1640 Hillcrest, Lawrence Kansas 66044(US)**
Inventor: **Pogany, Stefano A., 520 Louisiana, Lawrence Kansas 66044(US)**
Inventor: **Ringelsen, Cheryl, 202 North Normandy, Olathe Kansas 66061(US)**
Inventor: **Zentner, Gaylen M., 2628 Bardith Court, Lawrence Kansas 66044(US)**

(74) Representative: **Ahner, Francis et al, CABINET REGIMBEAU, 26, avenue Kléber, F-75116 Paris(FR)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



ACTORUM AG

## Description

There has been a long need in the field of drug delivery devices to have a drug released in the human body at the place where it is most therapeutically effective and to have said drug released in the body in a controlled manner over a long period of time.

There is art showing that poly(orthoester)s can be used as a matrix for drug release; however, there is no description in any of this art of the use of organic acids as catalysts to promote the polymer erosion in a controlled fashion.

US-A 4 322 323 (EP-A 0 052 916) describes drug delivery device incorporating surfactant as catalysts for the erosion of polymer.

US-A 4 489 056 (EP-A 0 100 424) discloses the use acid anhydrides as catalysts for the erosion of poly(orthoester)s. The patent discloses that the acid anhydride in an aqueous environment generates the corresponding acid. Acid anhydrides may in certain instances have the disadvantage, however, that they are reactive electrophiles that can acylate drugs containing a nucleophilic group, such as a hydroxyl, amino, carboxyl or sulfhydryl group. For example, when phthalic anhydride is used to catalyze the erosion of poly(orthoester)s, significant quantities of phthalic esters of hexane diol, one of the diols which are monomers of the poly(orthoester), are observed. Similarly when timolol, a drug with a hydroxyl functionality, is incorporated into a poly(orthoester) with phthalic anhydride, a significant amount of timolol phthalate ester is observed. Similar reaction products are not observed when free acids, which are not acylating agents, are used as the acid catalysts.

The release of norethindrone from poly(orthoester)s slabs has been described in the prior art. However, in this system a water-soluble salt such as sodium chloride and the like was incorporated into the polymer and the proposed mechanism for drug release in this case was osmotic imbibing of water, causing the matrix to swell and burst. The drug release was not controlled by polymer erosion but by a swelling process.

The present invention is directed to a controlled release device for the delivery of drugs or other biological beneficial substances which comprises:

(a) acid labile polymers, in particular a poly(orthoester) polymer, and
(b) an erosion catalyzing amount up to a maximum of 25 percent, by weight, based on (a), of at least one organic acid selected from the group consisting of carboxylic acids, sulfonic acids, phosphatidic acids, acidic phosphate esters, and mixtures thereof, incorporated within the matrix of said poly(orthoester).

The rate of release of therapeutically effective drug substance(s) or other beneficial substances (hereafter collectively referred to as biologically active agents) incorporated into or surrounded by such polymer/acid catalyst mixtures is determined by the erosion pattern of the polymer resulting in controlled drug release.

It has been noted that poly(orthoester)s polymer linkages herein described are unstable at acidic pH but relatively stable at neutral or basic pH. Thus, a carboxylic and/or sulfonic acid which is incorporated into the polymer matrix would catalyze the matrix erosion. If, in addition, a pharmaceutical or therapeutic agent(s) or drug(s) or biologically active agent(s) is incorporated in the matrix of the polymer, it can be seen that the biologically active agent(s) can be released at a predictable rate from the polymer matrix as the polymer matrix is eroded by action of the acid. Also the polymer/acid matrix could surround the drug or beneficial substance whereby the drug or substance will be released when the polymer/acid matrix coating is eroded. The expected mechanism for breakdown and erosion of the polymer can be shown by the following equations where R is as defined below.

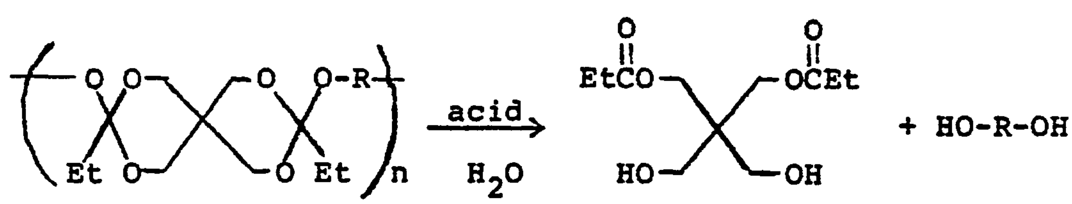


The rate of polymer matrix erosion and subsequently the rate of release of any drug(s) or biologically active agent(s) incorporated into or surrounded by the matrix can be controlled by choosing the proper acid or combination of acids. In selecting appropriate acids as catalysts the pKa, melting point, concentration, and hydrophobic character of the acids must be considered concomitantly for each poly(orthoester) system. For example, phthalic acid (melting point 230°) and the various phthalamic acid derivatives (melting points <100°C) discussed in the Examples section of the present invention have very similar first pKa's yet catalyze the erosion of the poly(orthoester) DETOSU-1,6-hexanediol:trans-cyclohexanedimethanol (65:35) differently; increased hydrophobicity and lower melting points of the acids correlated with improved catalytic behavior. In the same poly(orthoester) polymer, p-toluene sulfonic

acid (pKa<1; m.p. = 100°C) was an ineffective acid catalyst whereas phthalamic acid derivatives which had significantly higher pKa's were effective catalysts. Again, the increased hydrophobicity of the phthalamic acid derivatives relative to the p-toluene sulfonic acid played an important role. Thus it is clear that acid catalyst selection is sensitive to multiple features of the acid that can be manipulated to give the balance of pKa, melting point, and hydrophobicity to achieve the desired erosion rate of the polymer. Also, the concentration of the acid incorporated into the polymer matrix will control the rate of erosion of the polymer matrix and subsequently the rate of release of the drug substance incorporated therein. We have observed a linear relationship between the concentration of the acid incorporated and the rate of release of the drug substance and rate of erosion of the polymer when the drug or beneficial substance is incorporated in the polymer/acid matrix.

When the drug or beneficial substance is sorrounded or coated by the polymer/acid matrix, the drug or beneficial substance can be discharged when the matrix erodes in a predictable time frame.

In a more detailed description of the invention, the following types of polymers and acids are representative of those that can be used. Also there are described representative types of pharmaceuticals, therapeutic agents, biologically active agents or drugs or beneficial substances which can be incorporated into or surrounded by the matrix of said polymer to be released by reaction of the acid which catalyzes the matrix erosion.

Although any poly(orthoester) may be used, those described in U.S.-A 4 304 767 and US-A 4 093 709, are preferred.

Examples of poly(orthoester)s and other acid labile polymers disclosed in these two patents include:

1. Polymers of di(or higher functionality) keteneacetals and polyols which have a repeating mer unit of

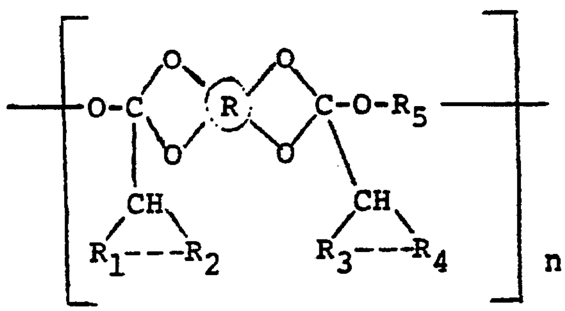

wherein n is an integer greater than 10; preferably 30 to 100. $R_1$ and $R_2$ are hydrogen or the same or different hydrocarbon groups and may be separate groups or may form parts of a cyclic group. Preferably $R_1$ is hydrogen and $R_2$ is methyl, ethyl, propyl, or the reverse. R is a quadrivalent organic grouping; preferably $C_5$ to $C_{30}$. $R_3$ and $R_4$ are hydrogen or the same or different hydrocarbon groups and may be separate groups or may form parts of a cyclic group. Preferably $R_3$ is hydrogen and $R_4$ is methyl, ethyl, propyl, or the reverse. $R_5$ is a hydrocarbon group which is the residue of a polyol $R_5(OH)_a$ wherein a is an integer equal to two or more, preferably 2 to 4, such polyol being a single molecular species or a mixture of molecular species; and wherein such linear chain may be crosslinked with other chains, and wherein R may be a single quadrivalent radical attached to all the interim acetal forming oxygen atoms, may be a spiro structure, may be an open chain aliphatic group, or may contain a carbocyclic group. For example, $R_5$ may be a linear six carbon chain or 1,4-dimethyl cyclohexane.

Additionally, $R_5$ may contain units that are alkylene or contain a carbocyclic group. Also included are polymers having the repeat units

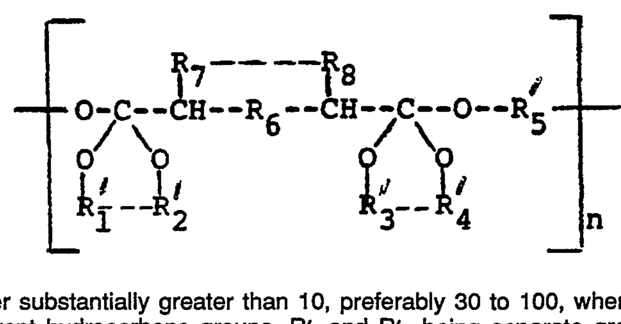

wherein n is an integer substantially greater than 10, preferably 30 to 100, wherein $R'_1$, $R'_2$, $R'_3$ and $R'_4$ are the same or different hydrocarbons groups, $R'_1$ and $R'_2$ being separate groups or parts of a cyclic group and $R'_3$ and $R'_4$ being separate groups or parts of a cyclic group. Preferably $R'_1$, $R'_2$, $R'_3$, and $R'_4$ are methyl, ethyl, propyl or combinations thereof. $R'_5$ is a hydrocarbon group which is the residue of

a polyol $R_5(OH)_a$, wherein a is an integer equal to two or more, preferably 2 to 4, and $R_5$ is as defined previously, such polyol being a single molecular species or a mixture of molecular species; $R_6$ is a valence bond or an hydrocarbon group; $R_7$ and $R_8$ are hydrogen or hydrocarbon groups which may be separate groups or may form parts of a cyclic group; and wherein such linear chains may be crosslinked to chains are also part of our invention. The group

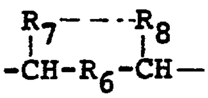

may be selected from the alkylene end groups containing a carbocyclic ring.

The types of acids which can be used in the invention are discussed below. Organic carboxylic or sulfonic acids may be used. Mixtures of various carboxylic acids and/or sulfonic acids may also be used.

Any organic carboxylic acid may be used. Examples include aliphatic acids which may be represented by the general formula R-COOH wherein R is a linear or branched hydrocarbon chain containing up to 30 carbon atoms, which may consist of aliphatic and aromatic branches. Also, R can be substituted at any carbon atom by the following groups: -OH, -OMe, OEt, -OR', -COOH, -COOMe, -COOEt, -COOR', $-CONH_2$, $-CONHR_1$, $-CONR_2Cl$, -F, $-NO_2$, -H where R' denotes an alkyl group containing up to thirty (30) carbon atoms.

Specific examples include acetic acid, propanoic acid, butanoic acid, pentanoic acid, dodecanoic acid, hexadecanoic acid, docosanoic acid, maleic acid, malic acid, lactic acid, atrolactic acid, mandelic acid, trichloroacetic acid, 2-hydroxy-2-phenyldodecanoic acid, 2,2-dichlorohexanoic acid, and 2,4-hexadienoic acid (sorbic acid).

Any aromatic carboxylic acid may be used. Aromatic carboxylic acids may be represented by the general formula aryl-$(COOH)_n$ wherein n is preferably 1 or 2 and "aryl" is a benzene ring or a naphthalene structure. Aryl can bear any of the following substituents at any position: -OH, -OMe, OEt, -OR', -COOH, -COOMe, -COOEt, -COOR', $-CONH_2$, -CONHR', $-CONR_2$, -Cl, -F, $-NO_2$, -H where R' (R-prime) denotes an alkyl group containing up to thirty (30) carbon atoms.

Specific examples include benzoic acid, p-dodecylbenzoic acid, p-isopropylbenzoic acid, 1-naphthoic acid, 2-naphthoic acid, 2-hydroxybenzoic acid (salicylic acid), acetylsalicylic acid (aspirin), and

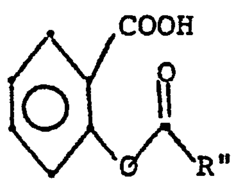

and

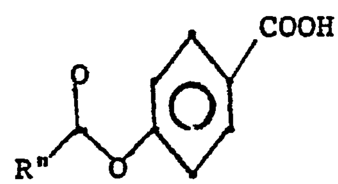

where R" (R-double-prime) = $C_{1-30}$, phthalic acid, phthalic acid monoesters, terephthalic acid, terephthalic acid monoesters, N-alkyl or N-dialkyl terephthalamic acids, and

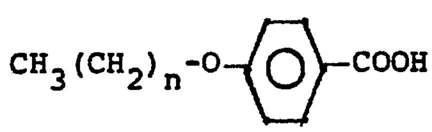

n = 1-30

specifically:
p-tetradecyloxybenzoic acid n = 13
p-octadecyloxybenzoic acid n = 17

Phthalic acid monoamides which are correctly named as N,N-dialkylphthalamic acids or N-alkyl phthalamic acids may also be used. Specific examples include N-butyl phthalamic acid, N-dodecyl phthalamic acid, N-methyl-N-dodecyl phthalamic acid, N-methyl-N-octadecyl phthalamic acid, and N-octadecyl phthalamic acid.

These phthalamic acids have the formula:

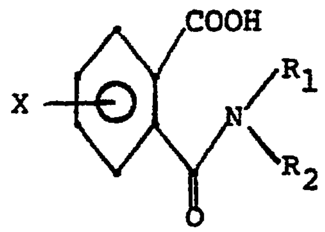

Where $R_1$ and/or $R_2$ = $C_{1-30}$ or H but both $R_1$ and $R_2$ cannot be hydrogen. X is $-NO_2$, halogen, or hydrogen.

Also included are polymers from which carboxylic or sulfonic acid groups are pendent.

Any sulfonic acid may be used. Sulfonic acids may be represented by the general formula $R-SO_3H$ wherein R can be aliphatic or aromatic and can contain up to 30 carbon atoms. Specific examples include benzenesulfonic acid, p-toluenesulfonic acid, dodecyl-benzene sulfonic acid, and dioctyl sulfosuccinate (free acid).

Phosphatidic acids and phosphate mono- and diesters can also be used as catalysts for polymer erosion. The phosphatidic acids have the general formulas:

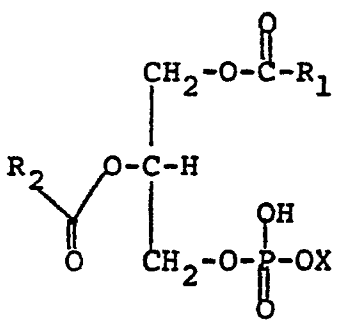

(A)

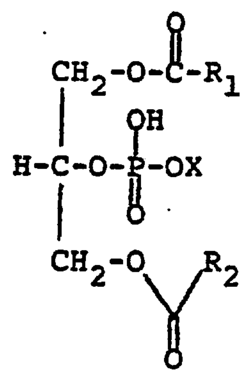

(b)

$R_1$ and $R_2$ can be the same or different, preferably alkyl radicals from $C_1$ to $C_{22}$. X is usually hydrogen or an alkyl radical from $C_1$ to $C_{22}$ but can also be

$$-CH_2-CH_2-\overset{\oplus}{N}(CH_3)_3.$$

Specific examples include dipalmitoyl phosphatidic acid, dilauroyl phosphatidic acid, dimyristoyl phosphatidic acid, dioleoyl phosphatidic acid, distearoyl phosphatidic acid, dibehenoyl phosphatidic acid, dicaproyl phosphatidic acid, didecanoyl phosphatidic acid, and dielaidoyl phosphatidic acid.

The phosphate monoesters have the general formula:

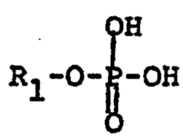

and the phosphate diesters have the general formula:

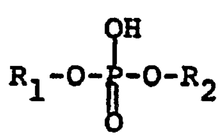

where $R_1$ and $R_2$ can be the same or different and are preferably essentially hydrocarbon radicals from $C_1$ to $C_{22}$.

Suitable drugs (therapeutics) and beneficial substances (biologically active agents) for incorporation into or to be surrounded by the polymer matrix to be used with this invention and to be released to an aqueous environment include without limitation, the following: pesticides, herbicides, germicides, biocides, algicides, rodenticides, fungicides, insecticides, antioxidants, plant growth promoters, fertilizers, plant growth inhibitors, preservatives, disinfectants, sterilization agents, catalysts, chemical reactants, fermentation agents, foods, food supplements, nutrients, cosmetics, drugs, vitamins, sex sterilants, fertility inhibitors, fertility promoters, air purifiers, microorganism attenuators, vaccines and other agents that benefit that environment of use.

In the specification and the accompanying claims, the term "drug" includes any physiologically or pharmacologically active substances that produce a localized or systemic effect or effects in animals, which term includes mammals, humans and primates. The term also includes domestic household, sport or farm animals such as sheep, goats, cattle, horses, chickens, turkeys and pigs, for administering to laboratory animals such as mice, rats and guinea pigs, and to fishes, to avians, to reptiles and zoo animals. The term "physiologically" as used herein denotes the administration of drug to produce normal levels and functions. The term "pharmacologically" denotes variations in response to amounts of drug including therapeutics. Stedman's Medical Dictionary, 1966, published by Williams & Wilkins, Baltimore, Md. The phrase drug formulation as used herein means the drug may be by itself, or the drug may be mixed with solutes, binders, dyes, mixtures thereof, and the like. The active drug that can be delivered includes inorganic and organic compounds without limitation, including drugs that act on the peripheral nerves, adrenergic receptors, cholinergic receptors, nervous system, skeletal muscles, cardiovascular, smooth muscles, blood circulatory system, synoptic sites, neuro-effector junctional sites, endocrine and hormone systems, immunological system, reproductive system, skeletal system, autocoid systems, alimentary and excretory systems, inhibitory of autocoids and histamine systems, those materials that act on the central nervous system such as hypnotics and sedatives, including pentobarbital sodium, phenobarbital, secobarbital, thiopental and mixtures thereof; heterocyclic hypnotics such as dioxopiperidines and glutarimides; hypnotics and sedatives such as amides and ureas, exemplified by diethylisovaleramide and α-bromoisovaleryl urea; hypnotic and sedative urethanes and disulfanes; psychic energizers such as isocoboxazid, nialamide, phenelzine, imipramine, amitryptyline hydrochloride, tranylcypromine and pargylene; and protryptyline hydrochloride, tranquilizers such as chloropromazine, promazine, fluphenzaine, reserpine, deserpidine, meprobamate, and benzodiazepines such as diazepam and chlordiazepoxide; anticonvulsants such as primidone, enitabas, phenytoin, ethyltion, pheneturide and ethosuximide; muscle relaxants and antiparkinson agents such as mephenesin, methocarbomal, cyclobenzaprine trihexylphenidyl, levodopa/carbidopa, and biperiden; antihypertensives such as α-methyldopa and L-β-3-4-dihydroxyphenylalanine, and pivaloyloxyethyl ester of α-methyldopa hydrochloride dihydrate; analgesics such as morphine, codeine, meperidine, nalorphine; antipyretics and anti-inflammatory agents such as aspirin, indomethacin, sodium indomethacin trihydrate, salicylamide, naproxen, colchicine, fenoprofen, sulindac, diflunisal, diclofenac, indoprofen and sodium salicylamide; local anesthetics such as procaine, lidocaine, maepaine, piperocaine, tetracaine and dibucane; antispasmodics and muscle contractants such as atropine, scopolamine, methscopolamine, oxyphenonium, papaverine; prostaglandins such as $PGE_1$, $PGE_2$, $PGF_{1\alpha}$, $PGF_{2\alpha}$ and PGA; antimicrobials and antiparasitic agents such as penicillin, tetracycline, oxytetracycline, chlorotetracycline, chloramphenicol, thiabendazole, ivermectin, and sulfonamides; antimalarials such as 4-aminoquinolines, 8-aminoquinolines and pyrimethamine; hormonal agents such as dexamethasone prednisolone, cortisone, cortisol and triamcinolone; androgenic steroids such as methyltestosterone, and fluoxmesterone; estrogenic steroids such as 17β-estradiol, α-estradiol, estriol, α-estradiol 3-benzoate, and 17-ethynyl estradiol-3-methyl ether; progestational steroids such as progesterone, 19-nor-pregn-4-ene-3,20-dione, 17-hydroxy-19-nor-17-α-pregn-5(10)-ene-20-yn-3-one, 17α-ethynyl-17-hydroxy-(5(10)-estren-3-one, and 9β,10α-pregna-4,6-diene-3,20-dione; sympathomimetic drugs such as epinephrine, phenylpropanolamine hydrochloride, amphetamine, ephedrine and norepinephrine; hypotensive drugs such as hydralazine; cardiovascular drugs such as procainamide, procainamide hydrochloride, amyl nitrite, nitroglycerin, dipyridamole, sodium nitrate and mannitol nitrate; diuretics such as chlorothiazide, acetazolamide, methazolamide, hydrochlorothiazide, amiloride hydrochloride and flumethiazide, ethacrynic acid, furosemide; antiparasitics such as bephenium, hydroxynaphthoate, dichlorophen and dapsone; and neoplastics such as mechlorethamine, uracil mustard, 5-fluorouracil, 6-thioguanine and procarbazine; β-blockers such as pindolol, propranolol, practolol, metoprolol, oxprenolol, timolol, timolol maleate, atenolol, alprenolol, and acebutolol; hypoglycemic drugs such as insulin, isophane insulin, protamine zinc insulin suspension, globin zinc insulin, extended insulin zinc suspension, tolbutamide, acetohexamide, tolazamide and chlorpropamide; antiulcer drugs such as cimetidine; nutritional agents such as ascorbic acid, niacin, nicotinamide, folic acid, choline, biotin, pantothenic acid, and vitamin $B_{12}$; essential amino acids; essential fats; eye drugs such as timolol, timolol maleate, pilocarpine, pilocarpine salts such as pilocarpine nitrate, pilocarpine hydrochloride, dichlorphenamide, atropine, atropine sulfate, scopolamine and eserine salicylate; histamine receptor antagonists such as cimetidine; and electrolytes such as calcium gluconate, calcium lactate, potassium chloride, potassium sulfate, sodium chloride, potassium fluoride, sodium fluoride, ferrous lactate, ferrous gluconate, ferrous sulfate, ferrous fumurate and sodium lactate; and drugs that act on α-adrenergic receptors such as clonidine hydrochloride.

Additional preferred drugs include quinoline and naphthyridine carboxylic acids and related compounds, such as 1-ethyl-6-fluoro-1,4-dihydro-4-oxo-7-(1-piperazinyl)-3-quinolinecarboxylic acid; 1-ethyl-1,4-dihydro-7-methyl-4-oxo-1,8-naphthyridine-3-carboxylic acid; 5-ethyl-5,8-dihydro-8-oxo-1,3-dioxolo-[4,5-g]quinoline-7-carboxylic acid; 8-ethyl-5,8-dihydro-5-oxo-2-(1-piperazinyl)pyrido[2,3-d]pyrimidine-6-carboxylic acid; 9-fluoro-6,7-dihydro-5-methyl-1-oxo-1H,5H-benzo[ij]quinoxolizine-2-carboxylic acid; 1-ethyl-1,4-dihydro-4-oxo-7-(4-pyridinyl)-3-quinolinecarboxylic acid; 1-ethyl-1,4-dihydro-4-oxo-[1,3]dioxolo[4,5-g]cinnoline-3-carboxylic acid; 9-fluoro-3-methyl-10-(4-methyl-1-piperazinyl)-7-oxo-2,3-dihydro-7H-pyrido[1,2,3-de][1,4]benzoxazine-6-carboxylic acid; 1-ethyl-6-fluoro-1,4-dihydro-7-(4-methyl-1-piperazinyl)-4-oxo-1,8-naphthyridine-3-carboxylic acid; 1-ethyl-6-fluoro-1,4-dihydro-7-(1-piperazinyl)-4-oxo-1,8-naphthyridine-3-carboxylic acid; 1-cyclopropane-6-fluoro-1,4-dihydro-4-oxo-7-(1-piperazinyl)-3-quinolinecarboxylic acid; 1-methylamino-6-fluoro-1,4-dihydro-4-oxo-7-(4-methyl-1-piperazinyl)-3-quinolinecarboxylic acid; 1-(4-fluoro-1-phenyl)-6-fluoro-1,4-dihydro-4-oxo-7-(1-piperazinyl)-3-quinolinecarboxylic acid; 1-(4-fluoro-1-phenyl)-6-fluoro-1,4-dihydro-4-oxo-7-(4-methyl-1-piperazinyl)-3-quinolinecarboxylic acid; 1-(4-fluoro-1-phenyl)-6-fluoro-1,4-dihydro-4-oxo-7-(1-piperazinyl)-1,8-naphthyridine-3-carboxylic acid; and 1-ethyl-6-fluoro-1,4-Dihydro-4-oxo-7-(3-ethylaminomethyl-1-pyrrolidinyl)-8-fluoro-3-quinolinecarboxylic acid.

Additional preferred drugs include drugs which affect the respiratory tract such as budesonide, enprofylline, tranilast, albuterol, theophylline, aminophylline, brompheniramine, chlorpheniramine, promethazine, diphenhydramine, azatadine, cyproheptadine, terbutaline, metaproterenol, and isoproterenol; drugs which are antidepressants such as amiflamine, alaproclate, doxepin, trazedone, maprotiline, zimelidine, fluvoxamine; antipsychotic drugs such as haloperidol, thioridazine, trifluoperazine, MK-0212, and remoxipride; sedative hypnotic and antianxiety drugs such as triazolam, temazepam, chlorazepate, alprazolam, diazepam, flurazepam, lorazepam, oxazepam, hydroxyzine, prazepam, meprobamate, butalbital, and chlorzoxazone; antiparkinson drugs such as benztropine and L-647,339; hormonal and steroidal drugs such as conjugated estrogens, diethylstilbesterol, hydroxy progesterone, medroxy progestrone, norethindrone, betamethasone, methylprednisolone, prednisone, thyroid hormone, levothyroxine and MK-0621; antihypertensive and cardiovascular drugs such as isosorbide dinitrate, digoxin, nadolol, disopyramide, nifedipine, quinidine, lidocaine, diltiazam, verapamil, prazosin, captopril, enalapril, lisinopril, metyrosine, felodipine, tocainide, mexiletine, mecamylamine, and metyrosine; diuretic drugs such as spironolactone, chlorthalidone, metolazone, triamterene, methyclothiazide, and indacrinone; antiinflammatory drugs such as ibuprofen, phenylbutazone, tolmetin, piroxicam, melclofenamate, auranofin, flurbiprofen and penicillamine; analgesic drugs such as acetaminophen, oxycodone, hydrocodone, and propoxyphene; antiinfective drugs such as cefoxitin, cefazolin, cefotaxime, cephalexin, nicarbazin, amprolium, ampicillin, amoxicillin, cefaclor, erythromycin, nitrofurantoin, minocyline, doxycycline, cefadroxil, miconazole clotrimazole, phenazopyridine, clorsulon, fludalanine, pentizidone, cilastin, phosphonomycin, imipenem, arprinocid, and foscarnet; gastrointestinal drugs such as bethanechol, clidinium, dicyclomine, meclizine, prochlorperizine, trimethobenzamide, loperamide, ranitidine, diphenoxylate, famotidine, metoclopramide and omeprazole; anticoagulant drugs such as warfarin, phenindione, and anisindione; and other drugs such as trientine, cambendazole, ronidazole, rafoxinide, dactinomycin, asparaginase, nalorphine, rifamycin, carbamezepine, metaraminol bitartrate, allopurinol, probenecid, diethylpropion, dihydrogenated ergot alkaloids, nystatin, pentazocine, phenylpropanolamine, phenylephrine, pseudoephedrine, trimethoprim, mevinolin, and ivermectin.

The above list of drugs is not meant to be exhaustive. Many other drugs will certainly work in the instant invention.

Examples of beneficial drugs are disclosed in Remington's Pharmaceutical Sciences, 16th Ed., 1980, published by Mack Publishing Co., Easton, Penna.; and in The Pharmacological Basis of Therapeutics, by Goodman and Gilman, 6th Ed., 1980, published by The MacMillian Company, London.

The drug can be in various forms, such as uncharged molecules, molecular complexes, pharmacologically acceptable salts such as hydrochlorides, hydrobromides, sulfate, laurylate, palmitate, phosphate, nitrite, borate, acetate, maleate, tartrate, oleate, and salicylate. For acid drugs, salts of metals, amines or organic cations, for example quaternary ammonium can be used. Derivatives of drugs such as esters, ethers and amides can be used alone or mixed with other drugs. Also, a drug that is water insoluble can be used in a form that is a water soluble derivative thereof to effectively serve as a solute, and on its release from the device, is converted by enzymes, hydrolyzed by body pH or other metabolic processes to the original form, or to a biologically active form. The agent can be in the form of a solution, dispersion, paste, cream, particle, granule, emulsion, suspension or powder. Also, the agent can be mixed with a binder, dispersant, emulsifier or wetting agent and dyes.

The amount of drug or beneficial substance incorporated into the polymer matrix will vary greatly depending on the particular drug, the desired therapeutic effect and the time span in which the polymer matrix is eroded to release the particular drug. Thus, there is no critical upper limit on the amount of drug incorporated in the polymer matrix and the lower limit will also depend on the activity of the drug and the time span for the erosion of the polymer and subsequently the drug release. Thus, it is not practical to define a range for the therapeutically effective amount of drug to be incorporated in the novel polymer matrices and acid combinations of the instant invention. However, in general the drug may be 0.001% to 70%, by weight, of the polymer/acid matrix by weight.

When the erodible polymer/acid matrix surrounds a drug formulation core as a coating or film to create a device the device can house from 0.05 ng to 5 grams or more of the drug formulation. The weight of the erodible polymer/acid matrix serving as the coating will vary depending on the surface area to be covered and the thickness needed to achieve the desired drug release profile. Typically the coat will be 10 to 1,000 microns thick although thicker and thinner fall within the invention.

The amount of acid incorporated into the polymer will be dependent upon the release duration of the drug or beneficial substance, and the particular acid used but would generally be in the range of slightly more than 0%, preferably 0.001% to a maximum of 25%, by weight, of the polymer by weight.

The drug or beneficial substance can be administered in various ways and shapes. For example, the polymer/acid/drug or beneficial substance can be incorporated into disc-shaped devices, rods or sheets for under the skin implantation, spherical shapes and the like. Those skilled in the art would realize that the invention can be incorporated in any shaped device for the particular application it is being used for.

The above described devices can be prepared by, for example:

1. Methods of preparation include:

1) Dissolution of components in solvent, evaporation of solvent, compression of matrix; 2) Mechanical milling of polymer, acid and drug or beneficial substance followed by compression; 3) Melt mixing of polymer, acid and drug or beneficial substance. In all cases, after mixing, standard pharmaceutical and plastics processing technology is used to make the dosage form.

In order to control the rate of release of the drug or beneficial substance in a programmable manner, one can laminate layers wherein the polymer, acid and drug or beneficial substance in each laminate layer are varied in concentration or contain different species of each component.

Multiple acids can be incorporated into the polymer to allow time variable or geometric considerations to be achieved. Additionally, the concentration of acid can be varied as a function of position in the matrix.

At least enough water must contact the device to react with the acid and the polymer to cause erosion. Water in excess of this amount will not materially effect the performance of the invention.

In order to better describe this invention, there follows examples illustrating the concepts of the invention.

EXAMPLE 1

960.0 mg of the acid labile polymer DETOSU-1,6-hexanediol: trans-cyclohexanedimethanol (65:35) were combined with 40.0 mg (0.12 mmoles) of the acid catalyst N-dodecyl phthalamic acid (m.p. 84-86°C) to make a final acid catalyst concentration of 4% N-dodecyl phthalamic acid, based on the polymer weight, then mixed for three minutes or until a homogeneous dispersion was achieved using a commercial mechanical shaker (WIG-L-BUG, Crescent Dental Manuf. Co.). The powdered mixture was placed in a Custom Scientific Mini Max Injection Molder pre-heated to 120-130°C, mixed for 1.5 minutes until the mixture was molten, then injected into a heated die (130°C) containing three cavities each 1 cm in diameter and three mm thick. The die was rapidly cooled to room temperature with dry ice. The discs were removed from the die. Each disc was placed in 200 ml of 0.05 M phosphate buffer, 37°C, pH 7.4, in standard USP dissolution vessels and agitated at 100 rpm using a DS500S stirrer (MEDIX Technologies, Inc.). Erosion of the discs was followed by assaying for one of the polymer's degradation products, 1,6-hexanediol, using an HP5880A gas chromatograph. The discs eroded at a zero-order rate of 3.52% per hour with a lag time of 1.9 hours (see Figure 1, curve A). Polymer containing no added acidic catalyst did not measurably erode over the same time course.

EXAMPLE 2

948.2 mg of the acid labile polymer DETOSU-1,6-hexanediol: trans-cyclohexanedimethanol (65:35) were combined with 51.8 mg (0.12 mmoles) of the acid catalyst N-methyl-N-octadecyl phthalamic acid (m.p. 70-72°C) to make a final acid catalyst concentration of 5.2% N-methyl-N-octadecyl phthalamic acid, based on polymer weight. The combination was mixed and injection molded as in Example 1; the same dissolution procedure and gas chromatographic assay was used to analyze the discs' erosion. The discs eroded at a zero-order rate of 4.2% per hour with a lag time of 5.1 hours (see Figure 1, curve B). Polymer containing no added acidic catalyst did not measurably erode over the same time period.

EXAMPLE 3

973.0 mg of the acid labile polymer DETOSU-1,6-hexanediol; trans-cyclohexanedimethanol (65:35) were combined with 27.0 mg (0.12 mmole) of the acid catalyst N-butyl phthalamic acid (m.p. 97-98°C) to make a final acid catalyst concentration of 2.7% N-butyl phthalamic acid, based on polymer weight. The combination was mixed and injection molded as in Example 1; the same dissolution procedure and gas chromatographic assay was used to analyze the discs' erosion. The discs eroded at a zero-order rate of 1.6% per hour with a lag time of 22.8 hours (see Figure 1, curve C). Polymer containing no added acidic catalyst did not measurably erode over the same time course.

EXAMPLE 4

980.0 mg of the acid labile polymer DETOSU-1,6-hexanediol: trans-cyclohexanedimethanol (65:35) were combined with 20.0 mg (0.12 mmoles) of the acid catalyst phthalic acid (m.p. 230°C) to make a final acid catalyst concentration of 2% phthalic acid, based on polymer weight. The combination was mixed and injection molded as in Example 1; the same dissolution procedure and gas chromatographic assay was used to analyze the discs' erosion. After 4 days, the discs were approximately 90% eroded. Polymer containing no added acidic catalyst did not measurably erode over the same time course.

EXAMPLE 5

961.0 mg of the acid labile polymer DETOSU-1,6-hexandiol: trans-cyclohexanedimethanol (65:35) were combined with 39.0 mg (0.12 mmoles) of the acid catalyst dodecylbenzenesulfonic acid to make a final acid catalyst concentration of 3.9% dodecylbenzenesulfonic acid, based on polymer weight. The combination was mixed and injection molded as in Example 1; the same dissolution procedure and gas chromatographic assay was used to analyze the discs' erosion. The discs eroded after approximately two days. Polymer containing no added acidic catalyst did not measurably erode over the same time course.

EXAMPLE 6

942.2 mg of the acid labile polymer DETOSU-1,6-hexanediol:trans-cyclohexanedimethanol (65:35) were combined with 57.8 mg (0.12 mmole) of the acid catalyst N-methyl-N-octadecyl-5-nitrophthalamic acid (m.p. 68-70°C) to make a final acid catalyst concentration of 5.8% N-methyl-N-octadecyl-5-nitrophthalamic acid, based on polymer weight. The combination was mixed and injection molded as in Example 1; the same dissolution procedure and gas chromatographic assay was used to analyze the discs' erosion. The discs eroded at a zero order rate of 9.9% per hour with a lag time of 0.61 hours (see Fig. 1, curve D). Polymer containing no added acidic catalyst did not measurably erode over the same time course.

## Claims

1. A controlled release device for the delivery of drugs or other biological beneficial substances which comprises:

(a) an acid labile polymer, which is a di (or a higher functionality) ketene acetals and polyols of the formula

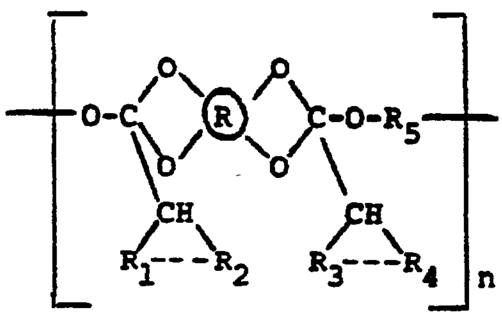

wherein n is an integer greater than 10; and

wherein $R_1$ and $R_2$ are hydrogen or the same or different hydrocarbon groups and may be separate groups or may form parts of a cyclic group; R is a quadrivalent organic grouping; $R_3$ and $R_4$ are hydrogen or the same or different hydrocarbon groups and may be separate groups or may form parts of a cyclic group; $R_5$ is a hydrocarbon group which is the residue of a polyol $R_5(OH)_a$ wherein a is an integer equal to two or more, such polyol being a single molecular species or a mixture of molecular species; and wherein such linear chain may be crosslinked with other chains, and wherein R may be a single quadrivalent radical attached to all the interim acetal forming oxygen atoms, may be a spiro structure, may be an open chain aliphatic group, or may contain a carbocyclic group and wherein additionally, $R_5$ may contain units that are alkylene or contain a carbocyclic group and including polymers having the repeat units

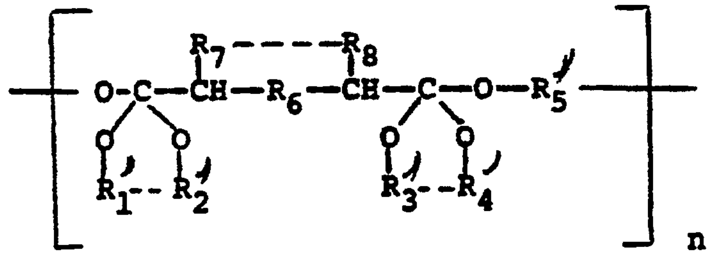

wherein n is an integer greater than 10; wherein $R'_1$, $R'_2$, $R'_3$ and $R'_4$ are the same or different hydrocarbon groups, $R'_1$ and $R'_2$ being separate groups or parts of a cyclic group and $R'_3$ and $R'_4$ being separate groups or parts of a cyclic group; $R'_5$ is a hydrocarbon group which is the residue of a polyol $R'_5(OH)_{a'}$ wherein a' is an integer equal to two or more, such polyol being a single molecular species or a mixture of molecular species; $R_6$ is a valence bond or an hydrocarbon group; $R_7$ and $R_8$ are hydrogen or hydrocarbon groups which may be separate groups or may form parts of a cyclic group; and wherein such linear chains may be crosslinked to chains and wherein the group

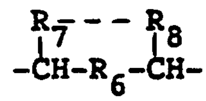

may be selected from the alkylene end group containing a carbocyclic ring, and
(b) an erosion catalyzing amount up to a maximum of 25 percent, by weight, based on (a), of at least one organic acid selected from carboxylic acids, sulfonic acids, phosphatidic acids, acidic phosphate esters, and mixtures thereof, incorporated within the matrix of said acid labile polymer.

2. The controlled drug release device of Claim 1, further comprising an effective amount up to 70 percent, by weight, based on (a) and (b), of a drug or other biologically beneficial substance incorporated within or surrounded by the matrix of said acid labile polymer.

3. The controlled drug release device of Claim 2, wherein the drug or biological beneficial substance is a protein drug, a desensitizing agent, a vaccine, an anti-infective, an antiallergenic, a steroidal anti-inflammatory, a decongestant, a miotic, an anticholinergic, a sympathomimetic, a sedative; a hypnotic, a psychic energizer, a tranquilizer, an androgenic steroid, an estrogen, a progestatinal agent, a humoral agent, an antipyretic analgesic, an antispasmotic, an antimalarial, an antihistamine, a cardioactive agent, a non-steroidal anti-inflammatory, an antiparkinsonian agent, an antihypertensive agent, a β-adrenergic blocking agent, a nutritional agent, a herbicide, a pesticide, a biocide, a fertilizer or an antifouling compound.

4. The controlled drug release device of Claim 1, wherein said organic acid is selected from carboxylic acid, sulfonic acid, phosphatidic acid or acid phosphate ester, N-dodecyl phthalamic acid, N-octadecyl phthalamic acid, N-methyl-N-octadecyl phthalamic acid, N-butyl phthalamic acid, terephthalamic acid and derivatives, phthalic acid, dodecylbenzenesulfonic acid and N-methyl-N-octadecyl-5-nitro-phthalamic acid.

5. The controlled drug release device of Claim 1, wherein said acid labile polymer is DETOSU-1,6-hexanediol/trans cyclohexanedimethanol.

6. A process for catalyzing the erosion of an acid labile polymer which is an acid labile polymer as disclosed in claim 1, comprising incorporating within the matrix of an acid labile polymer an erosion catalyzing amount up to a maximum of 25 percent by weight, based on the weight of said acid labile polymer, of at least one organic acid selected from carboxylic acids, sulfonic acids, phosphatidic acids, acidic phosphate esters and mixtures thereof.

7. The process for catalyzing the erosion of an acid labile polymer according to claim 6, wherein said organic acid is selected from carboxylic acid, sulfonic acid, phosphatidic acid or acidic phosphate ester, N-dodecyl phthalamic acid, N-octadecyl phthalamic acid, N-methyl-N-octadecyl phthalamic acid, N-butyl phthalamic acid, terephthalamic acid and derivatives, phthalic acid, dodecylbenzenesulfonic acid and N-methyl-N-octadecyl-5-nitrophthalamic acid.

**Patentansprüche**

1. Eine kontrollierte Ausgabeeinrichtung zur Abgabe von Arzneimitteln oder anderen biologisch wertvollen Stoffen, umfassend:

(a) ein säurelabiles Polymeres, nämlich di- (oder höherfunktionelle) Ketenacetale und Polyole der Formel

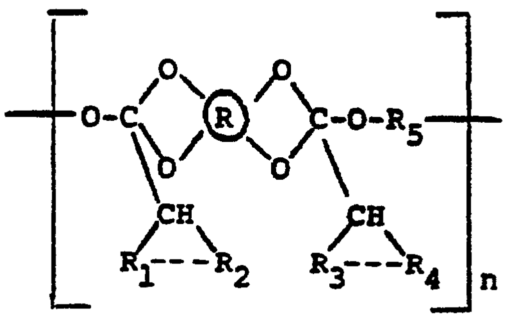

worin n eine ganze Zahl größer als 10 ist; und worin $R_1$ und $R_2$ Wasserstoff oder gleiche oder ungleiche Kohlenwasserstoffgruppen sind und gesonderte Gruppen sein können oder Teile einer zyklischen Struktur bilden können; R eine vierwertige organische Gruppierung ist; $R_3$ und $R_4$ Wasserstoff oder gleiche oder ungleiche Kohlenwasserstoffgruppen sind und gesonderte Gruppen sein können oder Teile einer zyklischen Gruppierung bilden können; $R_5$ eine Kohlenwasserstoffgruppe ist, welche der Rest eines Polyols $R_5(OH)_a$ ist, worin a eine ganze Zahl gleich zwei oder mehr ist, wobei das Polyol eine einzige molekulare Spezies oder ein Gemisch von molekularen Spezien ist; und worin eine solche lineare Kette vernetzt sein kann mit anderen Ketten, und worin R ein einziges vierwertiges Radikal ist, verknüpft an alle das Interimacetal bildenden Sauerstoffatome, eine Spirostruktur oder eine offenkettige aliphatische Gruppe sein kann oder eine carbozyklische Gruppe enthalten kann, und wobei zusätzlich $R_5$ Einheiten enthalten kann, die Alkylen sind oder eine carbozyklische Gruppe enthalten und Polymere der wiederkehrenden Einheit

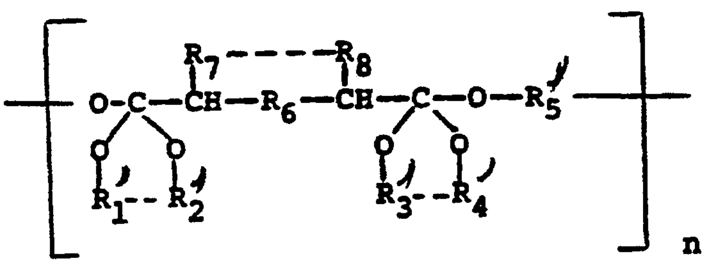

einschließen, worin n eine ganze Zahl von größer als 10 ist; worin $R'_1$, $R'_2$, $R'_3$ und $R'_4$ gleiche oder unterschiedliche Kohlenwasserstoffgruppen sind, $R'_1$ und $R'_2$ gesonderte Gruppen oder Teile einer zyklischen Gruppe und $R'_3$ und $R'_4$ gesonderte Gruppen oder Teile einer zyklischen Gruppe sind; $R'_5$ eine Kohlenwasserstoffgruppe ist, welche der Rest eines Polyols $R'_5(OH)_{a'}$ ist, a' eine ganze Zahl gleich zwei oder mehr ist, wobei ein solches Polyol eine einzige molekulare Spezies oder ein Gemisch molekularer Spezies ist; $R_6$ eine Valenzbindung oder eine Kohlenwasserstoffgruppe ist; $R_7$ und $R_8$ Wasserstoff oder Kohlenwasserstoffgruppen sind, die gesonderte Gruppen sind oder Teile einer zyklischen Gruppe bilden können; und worin solche lineare Ketten vernetzt sein können und worin die Gruppe

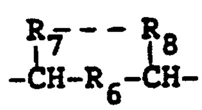

ausgewählt sein kann aus den Alkylenendgruppen, die einen carbozyklischen Ring enthalten, und

(b) eine erosionskatalysierende Menge bis zu einem Maximum von 25 Gew.%, bezogen auf (a), mindestens einer organischen Säure, ausgewählt aus Carbonsäuren, Sulfonsäuren, Phosphatidinsäuren, sauren Phosphatestern und Gemischen derselben, inkorporiert in die Matrix des säurelabilen Polymeren.

2. Kontrollierte Arzneimittelausgabeeinrichtung nach Anspruch 1, weiter enthaltend eine wirksame Menge von bis zu 70 Gew.%, bezogen auf (a) und (b), eines Arzneimittels oder einer anderen biologisch wertvollen Substanz, eingefügt in die Matrix des säurelabilen Polymeren oder von ihm umgeben.

3. Die kontrollierte Arzneimittelausgabeeinrichtung von Anspruch 2, worin das Arzneimittel oder die biologisch wertvolle Substanz ein Proteinmittel, ein desensibilisierendes Mittel, eine Vakzine, ein Antiinfektionsmittel, ein Antiallergikum, ein steroides entzündungshemmendes Mittel, ein Decongestantikum, ein miotisches Mittel, ein Anticholinergikum, ein Sympathomimetikum, ein Beruhigungsmittel; ein Hypnotikum, ein psychischer Energizer, ein Tranquilizer, ein androgenes Steroid, ein Estrogen, ein progestatinales Mittel, ein humorales Mittel, ein antipyretisches Analgetikum, ein Antispasmotikum, ein Antimalariamittel, ein Antihistamin, ein cardioaktives Mittel, ein nichtsteroides entzündungshemmendes Mittel, ein Antiparkinson-Mittel, ein antihypertensives Mittel, ein β-adrenergischer Blocker, ein Ernährungsmittel, ein Herbizid, ein Pestizid, ein Biozid, ein Düngemittel oder ein fäulnisverhinderndes Mittel ist.

4. Die kontrollierte Arzneimittelausgabeeinrichtung von Anspruch 1, worin die organische Säure ausgewählt wird aus Carbonsäure, Sulfonsäure, Phosphatidinsäure oder sauren Phosphatestern, N-Dodecylphthalaminsäure, N-Octadecylphthalaminsäure, N-Methyl-N-octadecylphthalaminsäure, N-Butylphthalaminsäure, Terephthalaminsäure und Derivaten, Phthalsäure, Dodecylbenzolsulfonsäure und N-Methyl-N-octadecyl-5-nitrophthalaminsäure.

5. Die kontrollierte Arzneimittelausgabeeinrichtung von Anspruch 1, worin das säurelabile Polymere DETOSU-1,6-hexandiol/transcyclohexandimethanol ist.

6. Verfahren zur Katalysierung der Erosion eines säurelabilen Polymeren, welches ein säurelabiles Polymere wie in Anspruch 1 definiert ist, bei dem man in die Matrix eines säurelabilen Polymeren eine erosionskatalysierende Menge von bis zu maximal 25 Gew.%, bezogen auf das Gewicht des säurelabilen Polymeren, mindestens einer organischen Säure, ausgewählt aus Carbonsäuren, Sulfonsäuren, Phosphatidinsäuren, sauren Phosphatestern und Gemischen derselben, einverleibt.

7. Verfahren zur Katalysierung der Erosion eines säurelabilen Polymeren nach Anspruch 6, worin die organische Säure ausgewählt wird aus Carbonsäure, Sulfonsäure, Phosphatidinsäure oder sauren Phosphatestern, N-Dodecylphthalaminsäure, N-Octadecylphthalaminsäure, N-Methyl-N-octadecylphthalaminsäure, N-Butylphthalaminsäure, Terephthalaminsäure und Derivaten, Phthalsäure, Dodecylbenzolsulfonsäure und N-Methyl-N-octadecyl-5-nitrophthalaminsäure.

## Revendications

1. Dispositif à libération réglée pour la distribution de médicaments et autres substances biologiques bénéfiques, qui comprend:

a) un polymère labile acide qui est un dicétèneacétal (ou d'une plus haute fonctionnalité) et un polyol répondant à la formule:

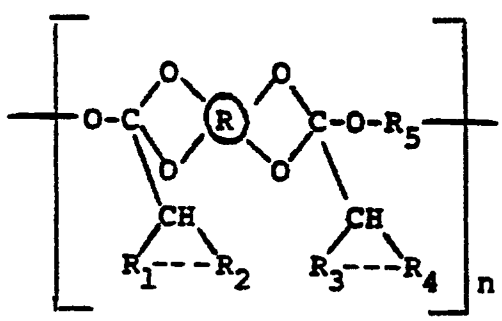

dans laquelle n est un nombre entier supérieur à 10; et $R_1$ et $R_2$ représentent chacun un atome d'hydrogène ou un radical hydrocarboné, qui peuvent être identiques ou différents et pouvant être des groupes séparés ou faire partie d'un groupe cyclique; R est un groupement organique quadrivalent; $R_3$ et $R_4$ représentent chacun un atome d'hydrogène ou un radical hydrocarboné qui peuvent être identiques ou différents et qui peuvent être des groupes séparés ou faire partie d'un groupe cyclique; $R_5$ est un radical hydrocarboné qui est le reste d'un polyol $R_5(OH)_a$ dans lequel a est un nombre entier de 2 ou plus, un tel polyol étant d'un seul type moléculaire ou un mélange de types moléculaires; la chaîne linéaire peut être réticulée avec d'autres chaînes; et R peut être un seul radical quadrivalent fixé à tous les atomes intérimaires d'oxygène formant l'acétal, peut être d'une structure spiro, peut être un radical aliphatique à chaîne ouverte ou peut contenir un groupe carbocyclique; et en outre, $R_5$ peut contenir des motifs qui sont alkylène ou contiennent un groupe carbocyclique et, notamment, des polymères comportant les motifs récurrents:

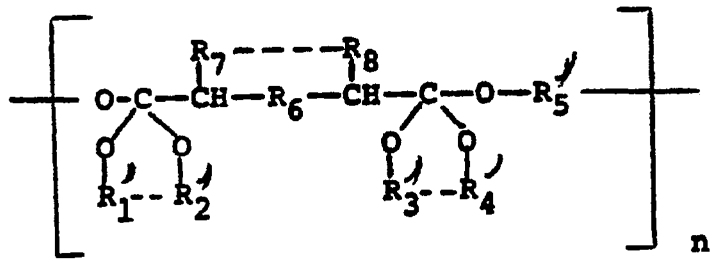

dans lesquels n est un nombre entier supérieur à 10; $R'_1$ $R'_2$, $R'_3$ et $R'_4$ sont des radicaux hydrocarbonés identiques ou différents, $R'_1$ et $R'_2$ étant des groupes séparés ou des parties d'un groupe cyclique et $R'_3$ et $R'_4$ étant des groupes séparés ou des parties d'un groupe cyclique; $R'_5$ est un radical hydrocarboné qui est le reste d'un polyol $R'_5(OH)_{a'}$ dans lequel a' est un nombre entier de 2 ou plus, un tel polyol étant d'une seule espèce moléculaire ou un mélange d'espèces moléculaires; $R_6$ est une liaison de valence ou un radical hydrocarboné; $R_7$ et $R_8$ sont des atomes d'hydrogène ou des radicaux hydrocarbonés qui peuvent être des groupes séparés ou faire partie d'un groupe cyclique; et ces chaînes linéaires peuvent être réticulées à des chaînes et le groupe:

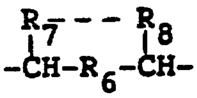

peut être un groupe alkylène ou un groupe contenant un noyau carbocyclique, et

b) une quantité catalysant l'érosion allant jusqu'à un maximum de 25% en poids par rapport à (a), d'au moins un acide organique choisi parmi les acides carboxyliques, les acides sulfoniques, les acides phosphatidiques, les phosphates acides et leurs mélanges, qu'on incorpore dans la matrice dudit polymère labile acide.

2. Dispositif à libération réglée de médicament, qui comprend en outre une quantité efficace allant jusqu'à 70% en poids par rapport à a) et b) d'un médicament ou autre substance biologiquement bénéfique qu'on incorpore dans la matrice dudit polymère labile acide ou qu'on entoure par cette matrice.

3. Dispositif de libération réglée de médicament selon la revendication 2, dans lequel le médicament ou la substance biologique est un médicament protéiné, un agent désensibilisant, un vaccin, un anti-infectieux, un antiallergénique, un anti-inflammatoire stéroidien, un décongestionnant, un myotique, un anticholinergique, un sympathomimétique, un sédatif; un hypnotique, un énergisant psychique, un tranquillisant, un stéroide androgène, un œstrogène, un agent progestatif, un agent humoral, un analgésique antipyrétique, un antispasmodique, un anti-malarial, un anti-hystaminique, un cardio-actif, un anti-inflammatoire non stéréodique, un anti-parkinsonien, un hypotenseur, un agent bloquant bêta-adrénergique, un agent nutritif, un herbicide, un pesticide, un biocide, un fertilisant ou un composé d'anti-encrassement.

4. Dispositif de libération réglée de médicament, selon la revendication 1, dans lequel ledit acide organique est choisi parmi l'acide carboxylique, l'acide sulfonique, l'acide phosphatidique ou un phosphate acide, l'acide N-dodécylphtalamique, l'acide N-octadécylphtalamique, l'acide N-méthyl-N-octadécylphtalamique, l'acide N-butylphtalamique, l'acide téréphtalamique et ses dérivés, l'acide phtalique, l'acide dodécylbenzènesulfonique et l'acide N-méthyl-N-octadécyl-5-nitro-phtalamique.

5. Dispositif de libération réglée de médicament selon la revendication 1, dans lequel ledit polymère labile acide est DETOSU-1,6-hexane-diol/trans-cyclohexane-diméthanol.

6. Procédé de catalyse de l'érosion d'un polymère labile acide qui est un polymère labile acide tel que décrit dans la revendication 1, qui consiste à incorporer dans la matrice d'un polymère labile acide une proportion catalysant l'érosion allant jusqu'à un maximum de 25% en poids par rapport audit polymère labile acide, d'au moins un acide organique choisi parmi les acides carboxyliques, les acides sulfoniques, les acides phosphatidiques, les phosphates acides et leurs mélanges.

7. Procédé de catalyse de l'érosion d'un polymère labile acide selon la revendication 6, dans lequel ledit acide organique est choisi parmi:
l'acide carboxylique, l'acide sulfonique, l'acide phosphatidique ou un phosphate acide, l'acide N-dodécylphtalamique, l'acide N-octadécylphtalamique, l'acide N-méthyl-N-octadécyl-phtalamique, l'acide N-butylphtalamique, l'acide téréphtalamique et ses dérivés, l'acide phtalique, l'acide dodécylbenzènesulfonique et l'acide N-méthyl-N-octadécyl-5-nitro-phtalamique.

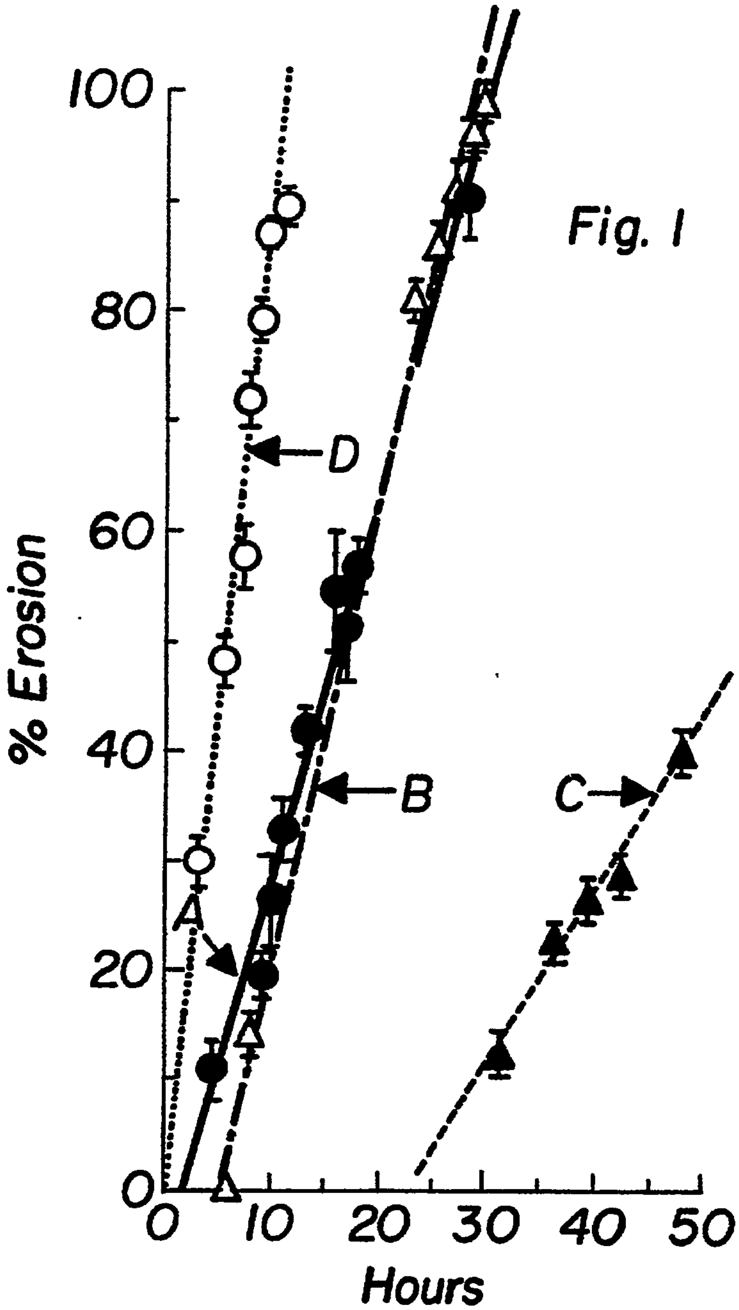


Fig. 1